# EUROPEAN PATENT APPLICATION

(11) **EP 2 380 873 A1**
(43) Date of publication of application: **26.10.2011**
(21) Application number: 10726798.1
(22) Date of filing: 05.01.2010
(51) Int. Cl.: C07C 303/44, C07C 303/32, C07C 309/06, C25B 3/06

(54) **PROCESS FOR PRODUCING PERFLUOROALKYLSULFONIC ACID SALT**

(30) Priority: 05.01.2009 JP 2009000408
(71) Applicant: Mitsubishi Materials Corporation, Chiyoda-ku Tokyo 100-8117 (JP); Mitsubishi Materials Electronic Chemicals Co., Ltd., Akita-shi Akita 010-8585 (JP)
(72) Inventor: HONDA, Tsunetoshi, Akita-shi Akita 010-8585 (JP); YATSUYANAGI, Hiroyuki, Akita-shi Akita 010-8585 (JP)
(74) Representative: Gille Hrabal Struck Neidlein Prop Roos
(86) International application number: PCT/JP2010/000013
(87) International publication number: WO 2010/076898

(57) **Abstract**

In this method for producing a perfluoroalkylsulfonic acid salt, there is provided a method for producing a perfluoroalkylsulfonic acid salt with a low fluorine content, which is a simple method to achieve a high yield,
wherein a solution is formed by adding, to at least one compound selected from perfluoroalkylsulfonic acid salts represented by a general formula RfSO₃•M (wherein Rf represents a perfluoroalkyl group of 1 to 4 carbon atoms, and M represents Li, Na or K), an organic solvent which is capable of dissolving the compound, and an adsorbent having a capacity to adsorb fluorine ions is added to the solution.

## Description

### [Technical Field]

The present invention relates to a method for producing a perfluoroalkylsulfonic acid salt, and particularly relates to a method for producing a perfluoroalkylsulfonic acid salt with a low fluorine content.

### [Background Art]

Conventionally, perfluoroalkylsulfonic acid salts represented by a general formula CₙF₂ₙ₊₁SO₃•M (where n represents an integer of 1 to 4 and M represents Li, Na, or K) have been suitably used as raw materials when producing perfluoroalkylsulfonic acids represented by a general formula CₙF₂ₙ₊₁SO₃H (where n represents an integer of 1 to 4) that are useful as synthesis catalysts during the production of drugs. In addition, perfluoroalkylsulfonic acid salts are also used as raw materials during the production of flame retardant, aromatic polycarbonate resin compositions (refer to Patent Document 1).

Here, in the flame retardant, aromatic polycarbonate resin compositions described in Patent Document 1, a high level of flame retardancy has been accomplished, in particular, due to the addition of perfluoroalkylsulfonic acid salts in which the level of fluorine ions has been reduced. Therefore, there has conventionally been a demand for perfluoroalkylsulfonic acid salts with reduced fluorine ion content.

Accordingly, a method for producing a perfluoroalkylsulfonic acid salt which enables the reduction of fluorine ion content has been proposed (Patent Documents 2 and 3). More specifically, the method for producing an alkali metal salt of perfluoroalkylsulfonic acid described in Patent Document 2 is a method to produce an alkali metal salt of perfluoroalkylsulfonic acid, in which the level of fluorine ions has been reduced, in the following manner: An alkali metal salt of perfluoroalkylsulfonic acid which is produced through, for example, a method of neutralizing the perfluoroalkylsulfonic acid with a carbonate, a hydroxide, or the like of an alkali metal is dissolved in ion-exchanged water, and after stirring for about 3 hours, for example, the resulting solution is cooled within a temperature range from 0°C to 40° C, followed by collection of precipitated crystals by filtration.

In addition, in the method for producing a potassium salt of perfluoroalkylsulfonic acid described in Patent Document 3, first, a gas prepared by electrolytic fluorination of perfluoroalkylsulfonyl fluoride represented by a general formula CₙF₍₂ₙ₊₁₎SO₂F is neutralized with a KOH aqueous solution to give an aqueous mixed solution composed of a potassium salt of perfluoroalkylsulfonic acid represented by a general formula CₙF(₂ₙ₊₁)SO₃K and potassium fluoride (KF). Subsequently, calcium hydroxide (Ca(OH)₂) is added to the obtained aqueous mixed solution to allow the reaction with potassium fluoride (KF), thereby forming calcium fluoride (CaF₂) and potassium hydroxide (KOH), followed by separation of calcium fluoride through filtration (first F removal step). Then, after neutralizing potassium hydroxide formed as a by-product with sulfuric acid, the aqueous mixed solution is concentrated, thereby yielding a mixture composed of a potassium salt of perfluoroalkylsulfonic acid (CₙF₍₂ₙ₊₁₎SO₃K) and potassium sulfate (K₂SO₄). At this point, the mixture contains several hundreds of ppm of fluorine (F).

Subsequently, concentrated sulfuric acid is added to the concentrated mixture of a potassium salt of perfluoroalkylsulfonic acid (CₙF₍₂ₙ₊₁₎SO₃K) and potassium sulfate (K₂SO₄) to allow for salt exchange, followed by distillation under reduced pressure to isolate perfluoroalkylsulfonic acid (CₙF₍₂ₙ₊₁₎SO₃H). At this point, the remaining fluorine (F) ions, for example, as KF are separated as hydrogen fluoride (HF) by distillation (second F removal step). Subsequently, perfluoroalkylsulfonic acid (CₙF₍₂ₙ₊₁₎SO₃H) is dissolved in water and then neutralized with potassium hydroxide (KOH) so as to achieve a pH from 7 to 8. Thereafter, by concentrating the resulting neutralized solution, a potassium salt of perfluoroalkylsulfonic acid with reduced fluorine ion content is produced.

### [Citation List]

### [Patent Document]

[Patent Document 1] Japanese Unexamined Patent Application, First Publication No. 2005-112973
[Patent Document 2] Japanese Unexamined Patent Application, First Publication No. Hei 1-268671
[Patent Document 3] Japanese Unexamined Patent Application, First Publication No. 2007-119458

### [Summary of Invention]

### [Technical Problem]

However, in the method for producing an alkali metal salt of perfluoroalkylsulfonic acid described in Patent Document 2, since fluorine ions are removed by dissolving the formed alkali metal salt of perfluoroalkylsulfonic acid in water followed by recrystallization, the poor yield has been a problem.

On the other hand, in the method for producing a potassium salt of perfluoroalkylsulfonic acid described in Patent Document 3, not only because it is time consuming to undergo a large number of steps, but also because the method for second fluorine removal step involves separation of hydrogen fluoride (HF) by distillation, there have been problems of complicated operations as well as corrosion of glass apparatuses. In addition, with respect to the demand for raw materials of the flame retardant, aromatic polycarbonate resin compositions described in Patent Document 1, there has been a risk of unsatisfactory reduction in the fluorine ion content.

The present invention takes the above circumstances into consideration, with an object of providing a method for producing a perfluoroalkylsulfonic acid salt with a low fluorine content, which is a simple method to achieve high yield.

### [Solution to Problem]

In order to achieve the above object, the present invention adopts the aspects described below.
[1] A method for producing a perfluoroalkylsulfonic acid salt characterized by forming a solution by adding, to at least one compound selected from perfluoroalkylsulfonic acid salts represented by a formula (1) shown below, an organic solvent which is capable of dissolving the compound, and adding an adsorbent having a capacity to adsorb fluorine ions to the aforementioned solution:

   RfSO₃•M (1)

   In the above formula (1), Rf represents a perfluoroalkyl group of 1 to 4 carbon atoms, and M represents any one of elements Li, Na and K.
[2] The method for producing a perfluoroalkylsulfonic acid salt according to the above aspect [1] **characterized in that** at least one compound selected from perfluoroalkylsulfonic acid salts represented by the above formula (1) contains at least one compound selected from fluorides represented by a formula (2) shown below as an impurity:

   MF (2)

   In the above formula (2), M represents any one of elements Li, Na and K.
[3] The method for producing a perfluoroalkylsulfonic acid salt according to the above aspect [1] or [2] **characterized in that** a mixed solution is formed by adding the aforementioned adsorbent to the aforementioned solution, and following stirring and filtration of the aforementioned mixed solution, the obtained filtrate is concentrated.
[4] A method for producing a perfluoroalkylsulfonic acid salt characterized by including: an electrolytic fluorination step in which a production gas mainly composed of perfluoroalkylsulfonyl fluoride represented by a formula (3) shown below is obtained; a gas absorption step in which a gas absorbing liquid containing at least one compound selected from perfluoroalkylsulfonic acid salts represented by a formula (5) shown below is obtained by reacting the aforementioned production gas with an alkaline aqueous solution containing a compound represented by a formula (4) shown below; a concentration step in which the aforementioned gas absorbing liquid is concentrated to obtain a mixture of at least one compound selected from perfluoroalkylsulfonic acid salts represented by the formula (5) shown below and a compound represented by a formula (6) shown below; a first purification step in which an organic solvent having a solubility of not more than 100 g/l for the compound represented by the formula (6) shown below is added to the aforementioned mixture, and the precipitated compound represented by the formula (6) shown below is separated by filtration, thereby obtaining a first filtrate having at least one compound selected from perfluoroalkylsulfonic acid salts represented by the formula (5) shown below dissolved therein; a second purification step in which an adsorbent having a capacity to adsorb fluorine ions is added to the aforementioned first filtrate to form a mixed solution, and the aforementioned mixed solution is stirred to adsorb and remove the compound represented by the formula (6) shown below, followed by filtration of the aforementioned mixed solution to obtain a second filtrate; and a concentration and recovery step in which the aforementioned second filtrate is concentrated and dried to obtain crystals of at least one compound selected from perfluoroalkylsulfonic acid salts represented by the formula (5) shown below:

   RfSO₂F (3)

   MOH (4)

   RfSO₃•M (5)

   MF (6)

   In the above formulae (3) to (6), Rf represents a perfluoroalkyl group of 1 to 4 carbon atoms, and M represents any one of elements Li, Na and K.
[5] The method for producing a perfluoroalkylsulfonic acid salt according to any one of the above aspects [1] to [4] **characterized in that** the aforementioned adsorbent contains either one or both of silica and alumina as a major component thereof.
[6] The method for producing a perfluoroalkylsulfonic acid salt according to any one of the above aspects [1] to [5] **characterized in that** the aforementioned organic solvent contains at least one solvent selected from the group consisting of alcohol-based solvents, ether-based solvents, ketone-based solvents and ester-based solvents.
[7] The method for producing a perfluoroalkylsulfonic acid salt according to any one of the above aspects [1] to [6] **characterized in that** a fluorine content is not more than 0.5 ppm.

### [Advantageous Effects of Invention]

The method of the present invention for producing a perfluoroalkylsulfonic acid salt includes a constitution in which a solution is formed by dissolving a perfluoroalkylsulfonic acid salt containing a fluoride as an impurity in an organic solvent, and an adsorbent is added to this solution. As a result, the impurity that does not dissolve in the organic solvent can be separated by filtration, and also the adsorbent can selectively adsorb and remove the fluoride that is dissolved in the organic solvent in small amounts. Therefore, a perfluoroalkylsulfonic acid salt with a low fluorine content can be easily produced with a high yield.

### [Description of Embodiments]

### <First embodiment>

A method for producing a perfluoroalkylsulfonic acid salt which is an embodiment of the present invention will be described below.
A method for producing a perfluoroalkylsulfonic acid salt according to the present embodiment is configured by including: a step (electrolytic fluorination step) in which a production gas mainly composed of perfluoroalkylsulfonyl fluoride represented by a formula (7) shown below is obtained; a step (gas absorption step) in which a gas absorbing liquid containing at least one compound selected from perfluoroalkylsulfonic acid salts represented by a formula (9) shown below is obtained by reacting the aforementioned production gas with an alkaline aqueous solution containing a compound represented by a formula (8) shown below; a step (concentration step) in which the aforementioned gas absorbing liquid is concentrated to obtain a mixture composed of at least one compound selected from perfluoroalkylsulfonic acid salts represented by the formula (9) shown below and a compound represented by a formula (10) shown below; a step (first purification step) in which an organic solvent having a solubility of not more than 100 g/l for the compound represented by the formula (10) shown below is added to the aforementioned mixture, and the precipitated compound represented by the formula (10) shown below is separated by filtration, thereby obtaining a first filtrate having at least one compound selected from perfluoroalkylsulfonic acid salts represented by the formula (9) shown below dissolved therein; a step (second purification step) in which an adsorbent having a capacity to adsorb fluorine ions is added to the aforementioned first filtrate to form a mixed solution, and the aforementioned mixed solution is stirred to adsorb and remove the compound represented by the formula (10) shown below, followed by filtration of the aforementioned mixed solution to obtain a second filtrate; and a step (concentration and recovery step) in which the aforementioned second filtrate is concentrated and dried to obtain crystals of at least one compound selected from perfluoroalkylsulfonic acid salts represented by the formula (9) shown below:

RfSO₂F (7)

MOH (8)

RfSO₃•M (9)

MF (10)

In the above formulae (7) to (10), Rf represents a perfluoroalkyl group of 1 to 4 carbon atoms, and M represents any one of elements Li, Na and K.

### (Raw material)

In the method for producing a perfluoroalkylsulfonic acid salt according to the present embodiment, an alkylsulfonyl fluoride represented by a formula (11) shown below can be used as an electrolytic material. The alkylsulfonyl fluoride can be readily produced by the fluorine substitution of an alkylsulfonyl fluoride using potassium fluoride or the like, as shown in a formula (12) shown below.

RSO₂F (11)

RSO₂Cl + KF → RSO₂F + KCl (12)

In the above formula (11) and the above formula (12), R represents an alkyl group of 1 to 4 carbon atoms. In other words, methylsulfonyl fluoride, ethylsulfonyl fluoride, propylsulfonyl fluoride and butylsulfonyl fluoride can be used as the alkylsulfonyl fluorides represented by the above formula (11) and the above formula (12).

### (Electrolytic fluorination step)

First, an electrolytic fluorination step is a step of obtaining a production gas mainly composed of a perfluoroalkylsulfonyl fluoride represented by the above formula (7). Here, in the above formula (7), Rf represents a perfluoroalkyl group of 1 to 4 carbon atoms. In other words, examples of the perfluoroalkylsulfonyl fluoride represented by the above formula (7) include trifluoromethylsulfonyl fluoride, pentafluoroethylsulfonyl fluoride, heptafluoropropylsulfonyl fluoride and nonafluorobutylsulfonyl fluoride.

More specifically, in the electrolytic fluorination step, for example, an alkylsulfonyl fluoride represented by the above formula (11) is used as a raw material, and this material is charged into an electrolytic cell together with hydrofluoric acid and electrolyzed under normal pressure in a nitrogen gas atmosphere. As a result, as indicated in a formula (13) shown below, a perfluoroalkylsulfonyl fluoride represented by the above formula (7) is produced by the fluorine substitution of alkyl groups in the alkylsulfonyl fluoride represented by the above formula (11).

RSO₂F + HF → RfSO₂F↑ + H₂↑ (13)

In the above formula (13), R represents an alkyl group of 1 to 4 carbon atoms, and Rf represents a perfluoroalkyl group of 1 to 4 carbon atoms.

It should be noted that as a side reaction, fluoroalkanes, such as tetrafluoromethane (CF₄), and sulfonyl difluoride (SO₂F₂) are formed due to the decomposition of raw materials, intermediates and the produced perfluoroalkylsulfonyl fluoride.

Here, the produced perfluoroalkylsulfonyl fluorides have low boiling points, and are consequently discharged outside the system from the electrolytic cell as gases together with hydrogen generated as a by-product, fluoroalkanes and sulfonyl difluoride obtained as decomposition products, nitrogen serving as a substitution gas, and hydrofluoric acid serving as an electrolytic solvent.

Here, the substitution gas is not limited to nitrogen, and an inert gas such as argon may be used. Although the conditions for electrolytic fluorination are not limited, it is preferably conducted at a temperature from -50°C to 50°C and more preferably from 0°C to 20°C.

### (Gas absorption step)

Next, a gas absorption step is a step in which a gas absorbing liquid containing at least one compound selected from perfluoroalkylsulfonic acid salts represented by the above formula (9) is obtained by reacting a perfluoroalkylsulfonyl fluoride represented by the above formula (7) which is a production gas with an alkaline aqueous solution containing a compound represented by the above formula (8).

Here, examples of the compounds represented by the above formula (8) include lithium hydroxide (LiOH), sodium hydroxide (NaOH) and potassium hydroxide (KOH). In addition, examples of the perfluoroalkylsulfonic acid salts represented by the above formula (9) include lithium trifluoromethylsulfonate, sodium trifluoromethylsulfonate, potassium trifluoromethylsulfonate, lithium pentafluoroethylsulfonate, sodium pentafluoroethylsulfonate, potassium pentafluoroethylsulfonate, lithium heptafluoropropylsulfonate, sodium heptafluoropropylsulfonate, potassium heptafluoropropylsulfonate, lithium nonafluorobutylsulfonate, sodium nonafluorobutylsulfonate and potassium nonafluorobutylsulfonate.

More specifically, in the gas absorption step, for example, the production gas discharged from the electrolytic cell in the electrolytic fluorination step described above is made to pass through a condenser at 0 to -40°C, thereby liquefying the accompanying hydrofluoric acid, followed by returning to the electrolytic cell. Since the gas discharged from the condenser outlet contains hydrogen fluoride that has not been liquefied in the condenser, the gas is preferably washed by gas-liquid contact with a shower of water or a low-concentration alkaline aqueous solution so as to remove the hydrofluoric acid, and then introduced into a gas absorption tower where the gas is brought into contact with an alkaline aqueous solution.

As the alkaline aqueous solution, an alkaline aqueous solution obtained by dissolving a compound represented by the above formula (8) is used. More specifically, an aqueous lithium hydroxide solution, an aqueous sodium hydroxide solution or an aqueous potassium hydroxide solution can be used. Note that in the present embodiment, potassium hydroxide is used as an example of the compound represented by the above formula (8).

In the gas absorption step, the production gas described above and the aqueous potassium hydroxide solution are allowed to make gas-liquid contact so that the perfluoroalkylsulfonyl fluoride contained in the production gas is absorbed in the aqueous potassium hydroxide solution. In other words, the gas absorption step, as shown in a formula (14) shown below, the perfluoroalkylsulfonyl fluoride contained in the electrolytically generated gas as the major component thereof reacts with potassium hydroxide and forms a potassium salt of perfluoroalkylsulfonic acid, which is then dissolved and absorbed in the liquid.

RfSO₂F + 2KOH → RfSO₃K + KF + H₂O (14)

On the other hand, fluoroalkanes that are by-products contained in the electrolytically generated gas are not absorbed in the aqueous potassium hydroxide solution and thus discharged outside the system. In addition, sulfonyl difluoride (SO₂F₂) that is also a by-product contained in the electrolytically generated gas is absorbed in the aqueous potassium hydroxide solution to form potassium sulfate (K₂SO₄) and potassium fluoride (KF). This potassium sulfate has a relatively low solubility of 7.35 g/100 g (0°C) in water, and when a potassium salt of perfluoroalkylsulfonic acid is also present in the solution, the solubility declines further and precipitates are readily formed by virtue of the salting out effect. Consequently, by separating the precipitated potassium sulfate by filtration, a gas absorbing liquid containing a potassium salt of perfluoroalkylsulfonic acid and potassium fluoride can be obtained.

It should be noted that in the gas absorption step, it is preferable to use an aqueous solution of potassium hydroxide having a concentration of 10% or more and to allow the absorption of production gas until the concentration of potassium hydroxide reaches less than 1%. In this case, the temperature of the aqueous potassium hydroxide solution (absorbing liquid) is kept between 40°C and 90°C, preferably between 50°C and 80°C, and the flow rate of circulation is adjusted to allow the gas-liquid contact such that the liquid-gas ratio between the aqueous potassium hydroxide solution and the amount of production gas to be introduced is 10 or more. By controlling the liquid-gas ratio to 10 or more within the temperature range described above, gas absorption can be continued until the concentration of potassium hydroxide reaches less than 1% while maintaining the gas absorption rate from 95 to 100%. In addition, when the concentration of potassium hydroxide is 40% or higher, the viscosity of the aqueous solution is increased, which makes it difficult to handle. Therefore, the concentration of the aqueous potassium hydroxide solution is preferably between 10% and 40%.

### (Concentration step)

Next, a concentration step is a step in which the obtained gas absorbing liquid described above is concentrated and dried to recover a mixture composed of at least one compound selected from the perfluoroalkylsulfonic acid salts represented by the above formula (9) and a compound represented by the above formula (10). Here, examples of the compounds represented by the above formula (10) include lithium fluoride (LiF), sodium fluoride (NaF) and potassium fluoride (KF).

More specifically, in the concentration step of the present embodiment, the obtained gas absorbing liquid described above is concentrated and dried to recover a mixture composed of a potassium salt of perfluoroalkylsulfonic acid and potassium fluoride.
For the concentration and drying of the gas absorbing liquid described above, commonly used techniques can be employed. A method of concentrating and drying while stirring under reduced pressure is particularly preferable since the obtained potassium perfluoroalkylsulfonate crystals have a low water content and thus formed into a fine powder, which is easy to handle.

Although conditions are not limited in the method of concentrating and drying while stirring under reduced pressure, the temperature is preferably between 30°C and 200°C and more preferably between 50°C and 150°C.

### (First purification step)

Next, a first purification step is a step in which an organic solvent having a solubility of not more than 100 g/l for the compound represented by the above formula (10) is added to the recovered mixture described above, and the precipitated compound represented by the above formula (10) is separated by filtration, thereby obtaining a first filtrate having at least one compound selected from the perfluoroalkylsulfonic acid salts represented by the above formula (9) dissolved therein.

As an organic solvent, any organic solvent can be used as long as it is capable of dissolving at least one compound selected from the perfluoroalkylsulfonic acid salts represented by the above formula (9) and also having a solubility of not more than 100 g/1, preferably not more than 10 g/1, and more preferably not more than 1 g/1, for the compound represented by the above formula (10).

Specific examples of the above-mentioned organic solvent include alcohol-based solvents such as methanol, ethanol, n-propanol, iso-propanol, n-butanol and tert-butanol; glycol-based solvents such as ethylene glycol, diethylene glycol, polyethylene glycol, propylene glycol, dipropylene glycol, tripropylene glycol, polypropylene glycol, 1,4-butanediol, 1,5-pentanediol, ethylene glycol monomethyl ether, ethylene glycol monoethyl ether, ethylene glycol monobutyl ether, diethylene glycol monomethyl ether, diethylene glycol monoethyl ether and tripropylene glycol monomethyl ether; ketone-based solvents such as acetone, methyl ethyl ketone and methyl isobutyl ketone; ether-based solvents such as dioxane, tetrahydrofuran and ethyl ether; ester-based solvents such as ethyl acetate, butyl acetate and isobutyl acetate; aromatic solvents such as benzene, toluene, xylene, methylnaphthalene and solvent naphtha; and polar solvents such as dimethylformamide (DMF), dimethyl sulfoxide (DMSO) and acetonitrile. These solvents may be used alone or two or more types thereof may be used in combination.

Although there are no limitations, the recovered mixture described above is preferably dissolved in the above-mentioned organic solvent so that the concentration thereof in the solution is from 0.1% by weight to 90% by weight, and more preferably from 10% by weight to 70% by weight. The temperature during stirring is preferably between 0°C and 200°C and more preferably between 0°C and 50°C.

In the present embodiment, it is particularly preferable to use alcohol-based solvents and ketone-based solvents in view of their relatively low boiling points and ease of handling. It should be noted that in the present embodiment, explanations are provided by using acetone as an example, which is a ketone-based solvent.

More specifically, in the first purification step of the present embodiment, acetone is added to the recovered mixture of the above-mentioned potassium salt of perfluoroalkylsulfonic acid and potassium fluoride, and the precipitated potassium fluoride is separated by filtration, thereby obtaining a first filtrate having the potassium salt of perfluoroalkylsulfonic acid dissolved therein. For the filtration of potassium fluoride, commonly used techniques can be employed. More specifically, for example, it is preferable to conduct filtration using a 1-µm PTFE (polytetrafluoroethylene) filter, following stirring at room temperature for 20 hours. As a result, a first filtrate in which a potassium salt of perfluoroalkylsulfonic acid having a fluorine content of 1% or less, that is, a purity of 99% is dissolved can be obtained.

Here, the term "fluorine content" used in the present embodiment refers to a value measured by the method for measuring the concentration of fluorine ions shown below. That is, a sample solution of the above-mentioned first filtrate is diluted by 50-fold in terms of mass with ion-exchanged water, and the concentration of fluorine ions is measured using a fluorine ion meter (Ti-5101, manufactured by Toko Kagaku Kenkyusho) which has been calibrated in advance with standard solutions, each containing 0.1 ppm and 0.01 ppm or 0.1 ppm and 1.0 ppm of fluorine ions. The fluorine content present in the sample can be determined based on the obtained fluorine ion concentration, and based on this result, the fluorine content in the potassium salt of perfluoroalkylsulfonic acid following the first purification step of the present invention can be calculated. Note that the limit of determination and the limit of detection in the present method are 0.5 ppm and 0.05 ppm, respectively.

### (Second purification step)

Next, a second purification step is a step in which an adsorbent having a capacity to adsorb fluorine ions is added to the recovered first filtrate described above to form a mixed solution, and this mixed solution is stirred to adsorb and remove the compound represented by the above formula (10), followed by filtration of the mixed solution to obtain a second filtrate.

As an adsorbent, any adsorbent can be used as long as it has a capacity to adsorb fluorine ions and is capable of adsorbing and removing the compound represented by the above formula (10) in the organic solvent described above. Specific examples thereof include activated carbon, activated carbon fibers, carbon molecular sieves, silica gel, activated alumina and zeolites. In the present embodiment, it is preferable to use an adsorbent having either one or both of silica and alumina as a major component, and it is particularly preferable to use a molecular sieve, which is one kind of zeolite, having silica and alumina as major components.

With respect to the amount of adsorbent to be added, it is preferable to add so that the amount is from 0.01 % by mass to 50% by mass, more preferably from 0.1% by mass to 10% by mass, relative to at least one compound selected from the perfluoroalkylsulfonic acid salts represented by the above formula (9). When the amount of adsorbent added is less than 0.01 % by mass, adsorption and removal of the compound represented by the above formula (10) cannot be conducted satisfactorily and therefore undesirable. On the other hand, when the amount of adsorbent added exceeds 50% by mass, the amount of adsorbed compound represented by the above formula (10) no longer changes, leading to an increase in cost due to the excessive addition, which is undesirable.

Stirring following the addition of adsorbent is preferably conducted, for example, at a temperature between -10°C and 100°C for a duration of 0.1 hours to 100 hours, and more preferably at a temperature between 0°C and 50°C for a duration of 1 hour to 50 hours.

For the filtration of the mixed solution described above, commonly used techniques can be employed. More specifically, for example, it is preferable to conduct filtration using a 0.2-µm PTFE filter, following stirring at room temperature for 2 hours. As a result, a second filtrate in which perfluoroalkylsulfonic acid salts having a fluorine content of not more than 0.5 ppm are dissolved can be obtained.

More specifically, in the second purification step of the present embodiment, first, 1 g of a molecular sieve (MS 13X) in the powder form is added as an additive to 10 g of an acetone solution, which is the recovered first filtrate, having a concentration of a potassium salt of perfluoroalkylsulfonic acid of 35%, thereby forming a mixed solution of 10% by mass. Subsequently, this mixed solution is stirred at room temperature for 2 hours to adsorb and remove the potassium fluoride (KF) remaining in the mixed solution. Then, this mixed solution is filtered with a 0.2-µm PTFE filter, thereby obtaining the second filtrate. When the fluorine content of this second filtrate was measured using a fluorine ion meter, it was 0.05 ppm or less (0.15 ppm or less on the basis of solid).

### (Concentration and recovery step)

Lastly, a concentration and recovery step is a step in which the recovered second filtrate described above is concentrated and dried to obtain crystals of at least one compound selected from the perfluoroalkylsulfonic acid salts represented by the above formula (9). For the concentration and drying of the second filtrate, commonly used techniques can be employed. A method of concentrating and drying while stirring under reduced pressure is particularly preferable since the obtained potassium perfluoroalkylsulfonate crystals are formed into a fine powder, which is easy to handle.

Although conditions are not limited in the method of concentrating and drying while stirring under reduced pressure, the temperature is preferably between 30°C and 200°C and more preferably between 50°C and 150°C.

The method of the present embodiment for producing a perfluoroalkylsulfonic acid salt includes a constitution in which a solution is formed by dissolving a potassium salt of perfluoroalkylsulfonic acid containing potassium fluoride as an impurity in acetone serving as an organic solvent, and a molecular sieve serving as an adsorbent is added to this solution. As a result, potassium fluoride that does not dissolve in acetone can be separated by filtration, and also the molecular sieve can selectively adsorb and remove the potassium fluoride that is dissolved in acetone in small amounts. Therefore, a potassium perfluoroalkylsulfonic acid salt having a fluorine content of not more than 0.5 ppm can be easily produced with a high yield.

### <Second embodiment>

Next, a method for producing a perfluoroalkylsulfonic acid salt which is a second embodiment of the present invention will be described.

A method for producing a perfluoroalkylsulfonic acid salt according to the present embodiment is a method in which a solution is formed by adding, to at least one compound selected from the perfluoroalkylsulfonic acid salts represented by the above formula (9), an organic solvent which is capable of dissolving the compound, and an adsorbent having a capacity to adsorb fluorine ions is added to the aforementioned solution. In addition, in the method for producing a perfluoroalkylsulfonic acid salt according to the present embodiment, it is preferable that at least one compound selected from the perfluoroalkylsulfonic acid salts described above contain at least one compound selected from the fluorides represented by the above formula (10) as an impurity. Moreover, it is preferable to form a mixed solution by adding the aforementioned adsorbent to the aforementioned solution, and to concentrate a filtrate obtained by stirring and filtration of the aforementioned mixed solution.

In terms of the adsorbent, the same as those described in the above first embodiment may be used.

### (Raw material)

In the present embodiment, the perfluoroalkylsulfonic acid salts produced by the production method other than the aforementioned electrolytic fluorination in the first embodiment can be used as raw materials. That is, the method for producing perfluoroalkylsulfonic acid salts according to the present embodiment is a method to produce perfluoroalkylsulfonic acid salts having a fluorine content of not more than 0.5 ppm from the perfluoroalkylsulfonic acid salt (with a fluorine content exceeding 0.5 ppm) in which the reduction of fluorine content is insufficient.

### (Production method)

More specifically, from the "first purification step" through the "second purification step" and the "concentration and recovery step" in the first embodiment described above can be applied to the production method of the present embodiment. Here, in the first purification step of the present embodiment, when the fluorine content of the above-mentioned perfluoroalkylsulfonic acid salts used as raw materials is low, there are cases where the compound represented by the above formula (10) does not precipitate even if the above-mentioned organic solvent is added. In such cases, the filtration process may be omitted. In addition, by conducting the second purification step and the concentration and recovery step following the first purification step, perfluoroalkylsulfonic acid salts having a fluorine content of not more than 0.5 ppm can be produced as in the first embodiment.

As described above, even when the perfluoroalkylsulfonic acid salts produced by the method other than the aforementioned production method according to the first embodiment are used as raw materials, a potassium salt of perfluoroalkylsulfonic acid having a fluorine content of not more than 0.5 ppm can be easily produced with a high yield.

### [Examples]

The effects of the present invention will be described below in more detail using a series of examples. However, the present invention is in no way limited by these examples.

### (Example 1)

First, through the electrolytic fluorination of heptafluoropropylsulfonyl fluoride (C₃H₇SO₂F), gases (C₃F₇SO₂F, SO₂F₂ and HF) produced from an electrolytic cell were absorbed in and reacted with an aqueous KOH solution, thereby obtaining a gas absorbing liquid. Subsequently, this gas absorbing liquid was concentrated to prepare a mixed salt having a composition of 60% potassium heptafluoropropylsulfonate (C₃F₇SO₃K), 20% potassium fluoride (KF) and 20% potassium sulfate (K₂SO₄).

Next, as a first purification step, 90 g of the above-mentioned mixed salt and 100 g of acetone were charged into a 300-ml flask, and following stirring at room temperature for 20 hours, the resulting mixture was filtered using a 1-µm PTFE filter, thereby obtaining 150 g of a 35% acetone solution containing C₃F₇SO₃K. Here, the fluorine content of the acetone solution measured using a fluorine ion meter was 35 ppm (100 ppm of fluorine content on the basis of solid). In addition, SO₄ ions in the acetone solution could not be detected by ion chromatography.

Then, as a second purification step, 0.1 g of a molecular sieve 13X powder (manufactured by Union Showa K.K.) was added as an adsorbent to 100 g of the obtained acetone solution described above, and following stirring at room temperature for 2 hours, the resulting mixture was filtered using a 0.2-µm PTFE filter, thereby obtaining 95 g of a 35% acetone solution containing C₃F₇SO₃K. Here, the fluorine content of the acetone solution measured using a fluorine ion meter was not more than 0.5 ppm (not more than the limit of determination). Further, the obtained acetone solution was concentrated to obtain 35 g of C₃F₇SO₃K crystals. In addition, the fluorine content thereof measured using a fluorine ion meter was not more than 0.5 ppm.

### (Example 2)

0.5 g of the alumina 60 powder (manufactured by Merck KGaA, Darmstadt, Germany) was added as an adsorbent to 100 g of the acetone solution obtained in the first purification step of Example 1 described above, and following stirring at room temperature for 2 hours, the resulting mixture was filtered using a 0.2-µm PTFE filter, thereby obtaining 95 g of a 35% acetone solution containing C₃F₇SO₃K. Here, the fluorine content of the acetone solution measured using a fluorine ion meter was not more than 0.5 ppm (not more than the limit of determination). Further, the obtained acetone solution was concentrated to obtain 35 g of C₃F₇SO₃K crystals. In addition, the fluorine content thereof measured using a fluorine ion meter was not more than 0.5 ppm.

### (Example 3)

0.5 g of the silica gel 60 powder (manufactured by Merck KGaA, Darmstadt, Germany) was added as an adsorbent to 100 g of the acetone solution obtained in the first purification step of Example 1 described above, and following stirring at room temperature for 2 hours, the resulting mixture was filtered using a 0.2-µm PTFE filter, thereby obtaining 95 g of a35% acetone solution containing C₃F₇SO₃K. Here, the fluorine content of the acetone solution measured using a fluorine ion meter was not more than 0.5 ppm (not more than the limit of determination). Further, the obtained acetone solution was concentrated to obtain 35 g of C₃F₇SO₃K crystals. In addition, the fluorine content thereof measured using a fluorine ion meter was not more than 0.5 ppm.

### (Example 4)

20 g of potassium trifluoromethanesulfonate (CF₃SO₃K) having a fluorine content of 30 ppm was dissolved in 80 g of acetone, and 0.2 g of the molecular sieve 13X powder (manufactured by Union Showa K.K.) was added thereto, followed by stirring at room temperature for 2 hours. Then, the resulting mixture was filtered using a 0.2-µm PTFE filter followed by concentration of the filtrate, thereby obtaining 20 g of CF3SO₃K crystals. The fluorine content thereof measured using a fluorine ion meter was not more than 0.5 ppm.

### (Example 5)

20 g of potassium nonafluorobutanesulfonate (C₄F₉SO₃K) having a fluorine content of 10 ppm was dissolved in 80 g of acetone, and 0.2 g of the molecular sieve 13X powder (manufactured by Union Showa K.K.) was added thereto, followed by stirring at room temperature for 2 hours. Then, the resulting mixture was filtered using a 0.2-µm PTFE filter followed by concentration of the filtrate, thereby obtaining 20 g of C₄F₉SO₃K crystals. The fluorine content thereof measured using a fluorine ion meter was not more than 0.5 ppm.

### (Example 6)

20 g of potassium heptafluoropropylsulfonate (C₃F₇SO₃K) having a fluorine content of 50 ppm was dissolved in 80 g of methanol, and 2.0 g of the molecular sieve 13X powder (manufactured by Union Showa K.K.) was added thereto, followed by stirring at room temperature for 2 hours. Then, the resulting mixture was filtered using a 0.2-µm PTFE filter followed by concentration of the filtrate, thereby obtaining 20 g of C₃F₇SO₃K crystals. The fluorine content thereof measured using a fluorine ion meter was not more than 0.5 ppm.

### (Comparative Example 1)

20 g of potassium heptafluoropropylsulfonate (C₃F₇SO₃K) having a fluorine content of 50 ppm was dissolved in 80 g of ion-exchanged water, and 20.0 g of the molecular sieve 13X powder (manufactured by Union Showa K.K.) was added thereto, followed by stirring at room temperature for 2 hours. Then, the resulting mixture was filtered using a 0.2-µm PTFE filter followed by concentration of the filtrate, thereby obtaining 15 g of C₃F₇SO₃K crystals. In addition, the fluorine content thereof measured using a fluorine ion meter was 12 ppm.

### (Comparative Example 2)

10.0 g of anhydrous magnesium sulfate (manufactured by Kanto Chemical Co., Inc.) was added as an adsorbent to 100 g of the acetone solution obtained in the first purification step of Example 1, followed by stirring at room temperature for 2 hours. Then, the resulting mixture was filtered using a 0.2-µm PTFE filter followed by concentration of the filtrate, thereby obtaining 18 g of C₃F₇SO₃K crystals. In addition, the fluorine content thereof measured using a fluorine ion meter was 49 ppm.

### [Industrial Applicability]

The method of the present invention for producing a perfluoroalkylsulfonic acid salt includes a constitution in which a solution is formed by dissolving a perfluoroalkylsulfonic acid salt containing a fluoride as an impurity in an organic solvent, and an adsorbent is added to this solution. As a result, impurity that does not dissolve in the organic solvent can be separated by filtration, and also the adsorbent can selectively adsorb and remove the fluoride that is dissolved in the organic solvent in small amounts. Therefore, a perfluoroalkylsulfonic acid salt with a low fluorine content can be easily produced with a high yield, which is industrially applicable.

## Claims

1. A method for producing a perfluoroalkylsulfonic acid salt comprising:
forming a solution by adding, to at least one compound selected from perfluoroalkylsulfonic acid salts represented by a formula (1) shown below, an organic solvent which is capable of dissolving the compound; and
adding an adsorbent having a capacity to adsorb fluorine ions to the solution:
RfSO₃•M (1)
wherein Rf represents a perfluoroalkyl group of 1 to 4 carbon atoms, and M represents any one of elements Li, Na and K.

2. The method for producing a perfluoroalkylsulfonic acid salt according to Claim 1,
wherein at least one compound selected from perfluoroalkylsulfonic acid salts represented by the above formula (1) contains at least one compound selected from fluorides represented by a formula (2) shown below as an impurity:
MF (2)
wherein M represents any one of elements Li, Na and K.

3. The method for producing a perfluoroalkylsulfonic acid salt according to Claim 1 or 2, wherein a mixed solution is formed by adding the adsorbent to the solution, and following stirring and filtration of the mixed solution, the obtained filtrate is concentrated.

4. A method for producing a perfluoroalkylsulfonic acid salt comprising:
an electrolytic fluorination step in which a production gas mainly composed of a perfluoroalkylsulfonyl fluoride represented by a formula (3) shown below is obtained;
a gas absorption step in which a gas absorbing liquid containing at least one compound selected from perfluoroalkylsulfonic acid salts represented by a formula (5) shown below is obtained by reacting the production gas with an alkaline aqueous solution containing a compound represented by a formula (4) shown below;
a concentration step in which the gas absorbing liquid is concentrated to obtain a mixture composed of at least one compound selected from perfluoroalkylsulfonic acid salts represented by the formula (5) shown below and a compound represented by a formula (6) shown below;
a first purification step in which an organic solvent having a solubility of not more than 100 g/l for the compound represented by the formula (6) shown below is added to the mixture, and a precipitated compound represented by the formula (6) shown below is separated by filtration, thereby obtaining a first filtrate having at least one compound selected from perfluoroalkylsulfonic acid salts represented by the formula (5) shown below dissolved therein;
a second purification step in which an adsorbent having a capacity to adsorb fluorine ions is added to the first filtrate to form a mixed solution, and the mixed solution is stirred to adsorb and remove the compound represented by the formula (6) shown below, followed by filtration of the mixed solution to obtain a second filtrate; and
a concentration and recovery step in which the second filtrate is concentrated and dried to obtain crystals of at least one compound selected from perfluoroalkylsulfonic acid salts represented by the formula (5) shown below:
RfSO₂F (3)
MOH (4)
RfSO₃•M (5)
MF (6)
wherein Rf represents a perfluoroalkyl group of 1 to 4 carbon atoms, and M represents any one of elements Li, Na and K.

5. The method for producing a perfluoroalkylsulfonic acid salt according to any one of Claims 1 to 4, wherein the adsorbent contains either one or both of silica and alumina as a major component thereof.

6. The method for producing a perfluoroalkylsulfonic acid salt according to any one of Claims 1 to 5,
wherein the organic solvent contains at least one solvent selected from the group consisting of alcohol-based solvents, ether-based solvents, ketone-based solvents and ester-based solvents.

7. The method for producing a perfluoroalkylsulfonic acid salt according to any one of Claims 1 to 6, wherein a fluorine content is not more than 0.5 ppm.
